# EUROPEAN PATENT APPLICATION

(11) **EP 4 327 788 A1**
(43) Date of publication of application: **28.02.2024**
(21) Application number: 23193242.7
(22) Date of filing: 24.08.2023
(51) Int. Cl.: A61F 5/058, A61F 5/37

(54) **SIZE-ADJUSTABLE CRANIAL AND PROTECTIVE HELMET**

(30) Priority: 26.08.2022 TR 202213402
(71) Applicant: ABC Tip Saglik Spor Malzemeleri Tekstil Turizm Insaat Sanayi ve Ticaret Limited Sirketi, Istanbul (TR)
(72) Inventor: KARAISMAILOGLU, CENGIZ, ISTANBUL (TR)
(74) Representative: Berkkam, Ayfer

(57) **Abstract**

The invention relates to a cranial and protective helmet for correction of skull deformities or for impact protection. The fact that the inner layers (2) of the helmet are of varying thicknesses and in varying quantities, that they can be put on and taken off optionally, and that the dimension / size can be enlarged by reducing the thickness or quantity as the skull grows, eliminates the need to design a helmet suitable for the growing skull structure. Thanks to the developed cranial and protective helmet, the need for taking measurements has been eliminated with a helmet suitable for mass production. Cost has been reduced with the developed helmet. Due to the very high cost of helmets available in the sector, the use of helmets in mild cases is generally low. With the reduction of helmet costs, it is expected that the use of helmets will increase at levels where the degree of skull deformity is mild. Thus, it will be seen that treatment effectiveness increases and better results are achieved. After determining a general outer mold according to normal infant head measurements, the thicknesses of the outer layer (1), inner layers (2) and intermediate layers (3) are determined. The adjustment mechanism (6) was used to adjust the size and dimension for each baby. Thus, there is no need to take measurements for each baby separately and to produce helmets in different sizes.

## Description

### Technical Field

The invention relates to a cranial and protective helmet for correction of cranial deformities or for damage protection.

### Prior Art

Cranial helmets are used to reshape the skulls of infants with deformational skull deformities. Cranial helmets are used for all cranial deformities and skull protection in infants with non-synostotic positional plagiocephaly, including those with plagiocephalic and brachycephalic deformities of the skull. These helmets are individually designed both in our country and in the world.

In order to correct various skull deformities and asymmetry seen in newborns, there are cranial helmets produced individually in the sector. In personalized cranial helmets, the newborn's skull is first molded. The molding process can be done in several different ways. The newborn skull is molded with plaster, which is the traditional method, or the newborn skull is scanned with the 3D scanning method and measurements are taken. After the newborn skull is measured, helmets are produced individually using various methods. Generally, 3D printers are used for helmet production. Apart from 3D printer, CNC router can be used for production. As the newborn's skull grows, it is usually measured again and molding and new helmets are produced.

Helmets are produced in two different ways, active and passive, in terms of structure and working principle. An active helmet applies an active constriction pressure to force the skull to grow more normally. These helmets are known as Dynamic Orthoplast Cranioplasty (DOC) helmets. Active DOC helmets must be custom fitted. They are time consuming to manufacture and need to be replaced as the baby's skull grows. As a result, DOC helmet treatment requires multiple clinic visits and incurs high costs for the patient. In addition, placing an astringent force on the growing brain is not considered physiologic by most doctors. Passive helmets, on the other hand, act as a barrier and molding in the deformed part of the skull, correcting the shape of the skull. This allows the skull to grow more normally. Unlike active molding, passive helmets provide a gradual and physiological correction of the skull shape.

In the known state of the art, the United States patent document US2012296249A1 discloses an orthosis helmet, methods of manufacture and use thereof. The helmet has an outer wall (712) and an inner wall (714). According to one embodiment, the helmet orthosis is placed on the patient's head and secured using a chin strap component or the like. The inner layer comprises a multi-walled laminated plastic film.

In the known state of the art, the United States patent document US2013289459A1 describes a cranial helmet that can be used for infants. The helmet generally comprises a hard outer shell and an inner liner. The inner liner may include one or more layers of foam or other compressible material. The outer layer of the helmet consists of two separable parts. These two parts are joined by a hook. In addition, the helmet includes sensors and a microcontroller.

In the known state of the art, the Polish patent document PL2203 85B1 refers to an orthopedic helmet for correcting the shape of the skull. According to the invention, the helmet is characterized in that an inner layer is placed under the outer layer. The outer layer consists of 2 parts. One of the advantages of the helmet is that it is a breathable helmet with low weight due to the honeycomb-shaped cavities. The fixing mechanism may include hook and loop fasteners, buckle type pins, eyelets, drawstring snaps, DD type connectors.

In the known state of the art, the United States patent document US 10695211B2 refers to an orthosis helmet for the correction of deformities in the head shape of infants and young children. The orthosis comprises two parts and a fastening means for securing the two parts together. It can be produced by 3D printer based on a scan of the user's head.

In the known state of the art, the United States patent document numbered US6423019B1 mentions a cranial remodeling orthosis used to prevent the growth of skull deformities. There is an outer layer, an inner layer, an intermediate layer and a pressure sensor in the intermediate layer.

Cranial helmets in the known state of the art have some disadvantages.
1) Modeling and production processes by taking measurements separately for each baby due to its personalized production
2) Necessity to take measurements again as the skull grows and to design a new helmet in accordance with the skull dimensions
3) Since it is produced individually, it takes time to produce and takes a long time to produce, causing patients to start treatment late. Therefore, it causes an increase in the deformity in the baby's head and prolongs the treatment period.
4) Experiencing problems such as sweating, heat exhaustion, heat-induced redness, bad odor in babies due to the lack of an intermediate layer between the inner layer and the outer layer to ensure air circulation
5) Usually carving, reshaping, or re-molding the inner layer to be single or two-piece and size adjustment.

In the known state of the art helmets, only special production helmet models are applied for the treatment of patients. Helmets used for correcting skull deformities and protecting the skull in exemplary patent documents need to be replaced continuously in case the skull grows. Continuing with the appropriate helmet during the treatment process is important for the correction of skull deformities. This leads to constant replacement of the helmet and an additional cost, especially in infants due to rapid growth. Therefore, there is a need for the development of a cranial and protective helmet that allows reshaping the deformity of the skull, whose dimensions can be adjusted as desired and can be applied to mass production.

### Objectives of the Invention

The object of the present invention is to realize a cranial and protective helmet that allows the correction of skull deformities.

Another object of the present invention is the realization of a cranial and protective helmet with an internal structure that is breathable and adjustable in various thicknesses.

Another object of the present invention is the realization of a cranial and protective helmet having a modular structure with layers that allow the helmet size to be adjusted at will.

Another object of the present invention is to realize a cranial and protective helmet which, thanks to its modular structure, can be designed to be suitable for different skull deformities and allows the use of different molds according to each type of deformity.

Another object of the present invention is the realization of a cranial and protective helmet that functions as a protective helmet by protecting the newborn skull against any external impact.

### Detailed Description of the Invention

The cranial and protective helmet realized to achieve the objects of the present invention is shown in the attached figures.

These figures;

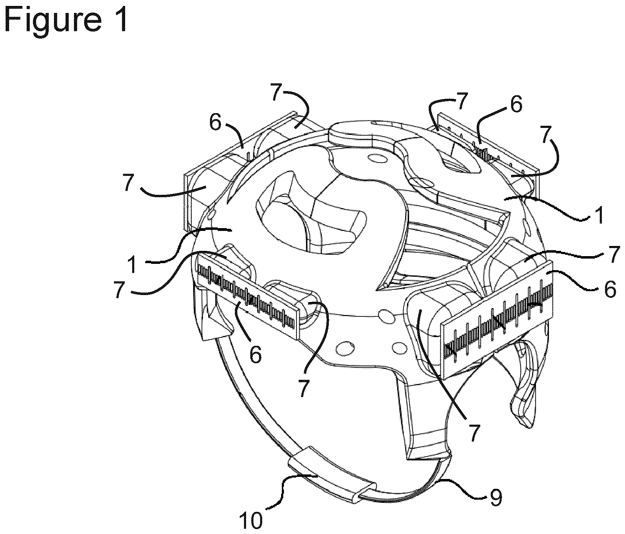

**Figure 1****:** Schematic view of the cranial and protective helmet.

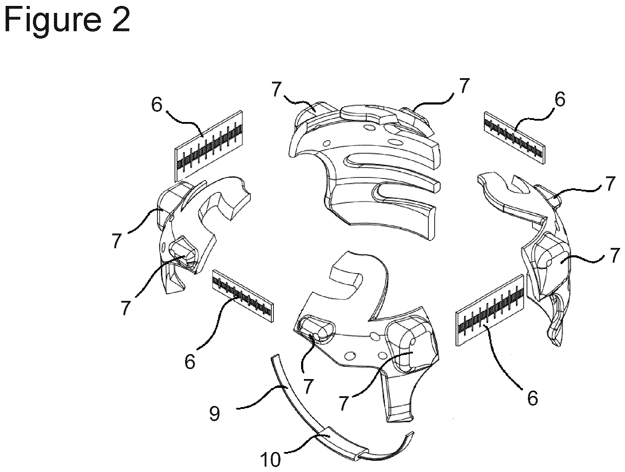

**Figure 2****:** Exploded view of parts of the cranial and protective helmet.

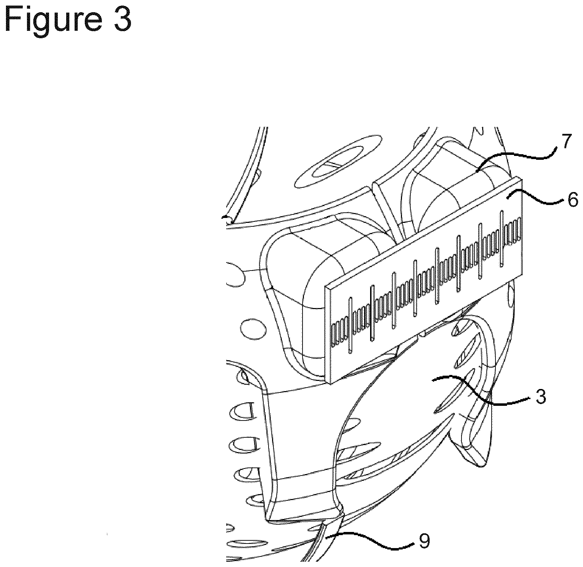

**Figure 3****:** View of the ear part of the cranial and protective helmet.

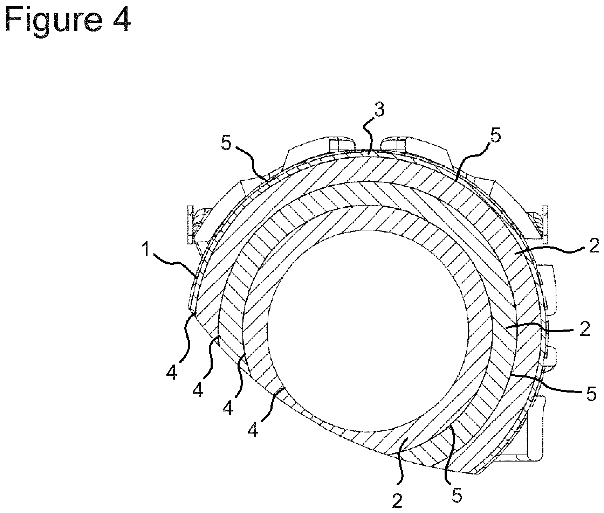

**Figure 4****:** Schematic view of the cross-section of the cranial and protective helmet.

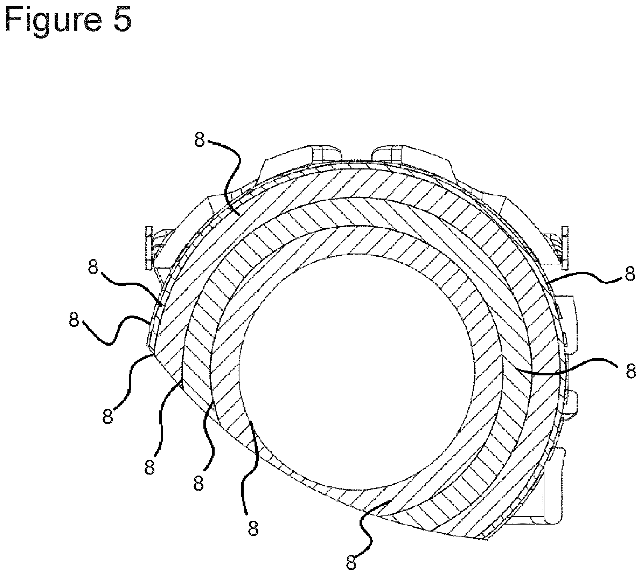

**Figure 5****:** Schematic view of the cross-section of the cranial and protective helmet.

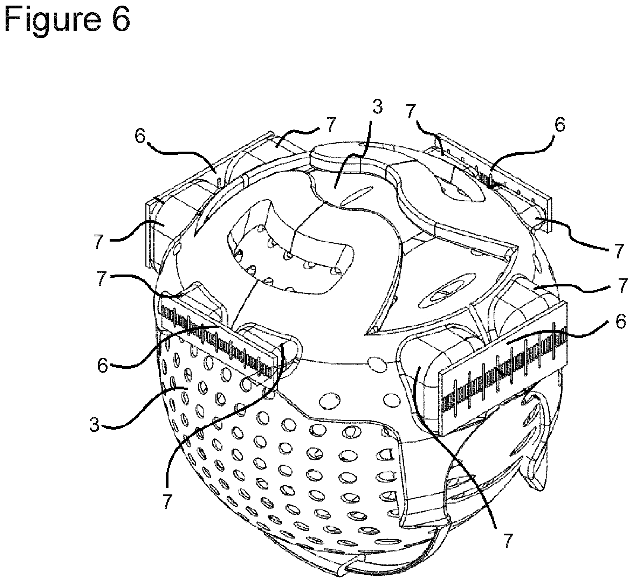

**Figure 6****:** Perspective view of the cranial and protective helmet.

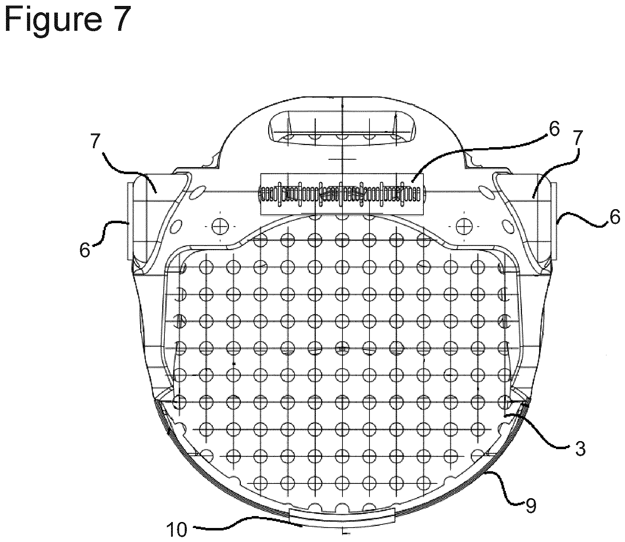

**Figure 7****:** Schematic frontal view of the cranial and protective helmet.

The parts in the figures are numbered one by one and the equivalents of these numbers are given below.
**1.** Outer layer
**2.** Inner layer
**3.** Intermediate layer
**4.** Laminated fabric
**5.** Fastening materials
**6.** Adjustment mechanism
**7.** Joining parts
**8.** Surface coating materials
**9.** Chin strap
**10.** Chin strap adjuster

The invention relates to a cranial and protective helmet for the correction of skull deformities and comprises;
- an outer layer (1) that acts as a mold for the helmet and has a modular and perforated structure,
- a perforated, breathable, soft, single or multi-layered inner layer (2) on the inner surface of the helmet, compatible with the outer layer (1),
- an intermediate layer (3) between the outer layer (1) and the inner layer (2), consisting of a mesh-shaped, perforated and thin plastic barrier, the thickness of which can be adjusted as desired,
- sweat-absorbing laminated fabric (4) that can be applied to the surface of the outer layer (1), inner layer (2) or intermediate layers (3),
- fastening materials (5) between the outer layer (1), the inner layer (2) or the intermediate layers (3), for fixing the outer layer (1) and the intermediate layer (3), the inner layer (2) and the intermediate layer (3), multiple inner layers (1) and the outer layer (1) and the inner layer (2) to each other,
- an adjustment mechanism (6) on the outer or inner surface of the helmet, which allows it to be assembled and secured, and makes it easier to adjust the size/size with millimetric adjustment marks,
- the modular parts of the outer layer (1) on the right, left, front and rear parts of the outer layer (1) of the helmet and the joining parts (7) that enable all parts to be joined,
- surface coating materials applied to the outer layer (1), inner layer (2) or intermediate layer (3) of the helmet with moisturizing effect, antibacterial and anti-microbial properties, or to prevent bad odor formation (8),
- chin strap (9), which is attached to the outer layer (1) and allows the helmet to be secured to the chin when the helmet is worn,
- chin strap adjuster (10) on the chin strap (9), which allows adjustment of the chin strap (9)
   parts.

The helmet subject to the invention acts as a mold for the newborn's head to grow as it should be normal without any force and pressure on the newborn's head. Since the dislocated parts of the newborn's skull will come into contact with the helmet and act as a barrier, the growth of the skull will tend to grow towards the flattened parts and the hollow parts that do not come into contact with the helmet because they are flattened. As a result, the skull will take the shape of the helmet as the skull grows towards the hollow parts. This is how the ideal skull shape will be achieved.

Based on the skull measurements of a normal healthy baby, the outer layer (1) is designed in computerized design programs. The outer layer (1) can be designed modularly as single or multi-part. Molds are obtained according to the design. The outer layer (1) is obtained by mass production methods such as injection printing. The outer surface of the outer layer (1) can be covered with a pattern if desired. The outer layer (1) is designed as single or multi-part, in different models suitable for different skull deformities (for plagiocephaly, brachycephaly, scaphocephaly and all skull deformities), modularly in computerized design programs. The thickness of the outer layer (1) can be produced in desired ratios. It can preferably be produced in the range of 2-8 mm or other thicknesses. The injection mold is designed in accordance with the outer layer (1) design and the outer layers (1) can be produced by injection molding method or other molding methods and mass production methods. Cranial helmets can be produced on mass production machines such as plastic injection molding machines. In the production of outer layers (1), a film layer, pattern layer, metal or polyurethane derivatives can be placed inside in injection molding.

The inner layer (2) is designed using computerized design programs. By designing cutting molds in accordance with the inner layers (2), inner layers (2) can be obtained by laser cutting or other mass production methods. The outer layer (1) and inner layer (2) are joined to each other with fastening materials (5) such as double-sided hook and loop fasteners, buckles and rivets. The inner layer (2) can be removed and reused after washing. The size can be adjusted by increasing or decreasing the number or thickness of the inner layers (2). Inner layers (2) can be produced with holes if desired. Holes can be in desired sizes. Air circulation is realized thanks to the holes in the inner layer (2). Inner layers (2) can be laminated with laminated fabric (4). Sweat that may occur due to newborns wearing the helmet for a long time is absorbed by laminated fabrics (4). This prevents the formation of a moist environment on the newborn skull. It provides a comfortable environment for the newborn and facilitates the newborn's adaptation to the helmet. The design of the inner layers (2) is designed in computerized design programs to be compatible with the outer layer (1) designs. There may be 3 or more or less inner layers (2) with different thicknesses in the range of 2-12mm or in appropriate ranges. It can be single layer or multi-layer. Inner layers (2) may be removable inside the helmet. Inner layers (2) may be laminated with laminated fabric (4). The inner layers (2) can be attached and detached from the intermediate layer (3) or the outer layer (1) with double-sided velcro, hook and loop fasteners, snaps and other suitable materials and devices. Inner layers (2) can be washed if desired. Inner layers (2) can have a perforated structure. Hole sizes can be in desired ranges. Inner layer (2) can be single or multi-layered. The inner layers (2) can be attached and detached to each other with double-sided hook and loop fasteners, velcro, snap fasteners and other suitable materials and apparatus. The size of the helmet can be adjusted by the thickness and quantity of the inner layer (2). For newborns from 4 months to 18 months, the size can be adjusted by increasing or decreasing the number of inner layers (2). Or again, the size can be adjusted by changing the thickness of the inner layer (2). In this way, the need to take measurements for each newborn using plaster or 3D scanning method is eliminated. At the same time, mass production is provided with the molding method, leaving personalized production. The helmet can be adjusted according to the head of newborn babies. With the developed helmet, a modular structure that can be easily adjusted in dimension and size is obtained. If desired, shaping is supported by placing all metal and plastic materials that can be shaped inside the inner layers (2) or adjustment mechanisms (6).

There may be an optional intermediate layer (3) between the inner layer (2) and the outer layer (1). The intermediate layer (3) may be in the form of a perforated mesh. The holes can be of the desired size. It can be attached and removed if desired. In this way, the newborn head can breathe. Sweating, heat exhaustion, heat-related redness, bad odor formation are prevented. The intermediate layer (3) is also designed in computerized design programs. The injection mold is designed and can be produced by injection printing or other mass production methods. There may optionally be an intermediate layer (3) between the outer layer (1) and the inner layer (2). The intermediate layer (3) can have a perforated or mesh structure. Hole sizes can be in desired ranges. The thickness of the intermediate layer (3) can be adjusted in 2-12 mm or in the desired appropriate ranges. Interlayer (3) is designed in computerized design programs. Injection mold is designed in accordance with the design of the intermediate layer (3) and using the designed injection mold, intermediate layers (3) can be produced by injection molding method or other molding methods and other mass production methods. The intermediate layer (3) can be optionally attached and detached from the outer layer (1) by means of screws, buckles, plug-in and plug-out puzzle mechanisms, etc. The intermediate layer (3) can be assembled between the inner layer (2) and the outer layer (1) with the help of fastening materials (5). The outer layer (1) parts can be assembled to each other with the help of fastening materials (5) such as velcro, straps, adjustment buckles, locking mechanisms, dimension adjustment mechanisms, rivets and adjustment mechanisms (6). The outer layers (1) can also be adjusted for size, dimension and tightness at the connection points with each other. Due to the prolonged wearing of the helmet on the newborn's head, redness, bad odor and a small amount of irritation may occur in some cases. To prevent these, microcapsules or nanoparticles made of lanolin and similar moisturizing or regenerating ingredients can be applied to the outer layer (1), inner layers (2) or intermediate layers (3). As the newborn wears the helmet, the microcapsules or nanoparticles disintegrate over time due to contact and temperature, providing moisture to the newborn's head and reducing irritation. To prevent bad odor, microcapsules or nanoparticles synthesized from plant extracts or suitable ingredients can be applied to the outer layer (1), inner layers (2) or intermediate layers (3). As the newborn wears the helmet, the microcapsules or nanoparticles explode over time, releasing fragrances and preventing bad odors. If desired, silver, copper, chitosan or similar microcapsules or nanoparticles can be applied to the inner layers (2) to prevent bacterial growth and prevent bad odor. Microcapsules or nanoparticles can be used on demand. Due to the removability of the inner layers (2) and the intermediate layer (3) and thanks to the adjustment mechanism (6), the helmet can be used without the need for a second measurement or a second helmet for size adjustment.

The design and sizing of the outer layer (1), inner layer (2) and intermediate layer (3) in accordance with the skull of a baby with normal skull measurements by using international standards and girl-boy percentile table are made in computerized design programs.

Each part can be assembled with materials such as Velcro, straps, hook and loop, or with plastic, metal or other suitable materials using the size adjustment mechanism (6). It can be easily attached and detached from these points when desired. With apparatus such as Velcro, straps, hook and loop, adjustment mechanism (6), millimeter size adjustments can be made. Thus, the size and fit of the helmet can be adjusted. In addition, if desired, a chin strap (9) can be added for fixation.

The following steps are followed in the production process and can be easily adapted to mass production by applying other mass production approaches.
1) Dimensions are determined in accordance with normal, healthy baby skull measurements. Dimensional measurement calculations are made for the outer layer (1), inner layer (2) and intermediate layers (3) of the helmet. Layer thicknesses are determined and layer thicknesses are adjusted according to the monthly growth rates of the newborn.
2) The outer layer (1) is designed in computerized design programs as single or multi-part, in different models suitable for different skull deformities, and modular. The thickness of the outer layer (1) can be in desired proportions. It can preferably be in the range of 2-8 mm or other thicknesses. Injection mold can be designed in accordance with the outer layer (1) design and the outer layers can be produced by injection molding method using the designed injection mold. Other molding and mass production methods can also be used in the production method. The outer surface of the outer layer (1) can be covered with a pattern if desired. For pattern coating, other suitable methods such as water printing can be used.
3) The inner layers (2) can be in different numbers and with different thicknesses in the range 2-12 mm or in appropriate ranges. Inner layer (2) can be single layer or multi-layer. It is such that it can be inserted and removed inside the helmet. The fabric may be laminated fabric (4). The inner layers (2) can be attached to the intermediate layer (3) or the outer layer (1) or the inner layers (2) can be attached and detached from each other with suitable fastening materials (5) such as double-sided velcro, hook and loop, snap fasteners. The inner layers (2) can be washable and perforated. For newborns from 4 months to 18 months of age, the helmet can be sized by increasing or decreasing the number of inner layers.
4) There may optionally be an intermediate layer (3) between the inner layers (2) and the outer layer (1). The intermediate layer (3) may have a perforated or mesh structure. The intermediate layer (3) can be 2-12 mm or in the desired suitable ranges.
5) Each part is assembled to each other using the size adjustment mechanism (6) with fastening materials (5) such as velcro, straps, hook and loop fasteners (5) that can be adjusted in length, extended and narrowed. The size and fit of the helmet can be adjusted.

In case of demand, raw materials that are shaped by temperature are produced in layers by methods such as injection molding. This layer is then heated and placed on the head model to take the shape of a normal baby's head and helmets are produced by shaping the raw material in this way.

The average size is taken from the percentile curves and the helmet is reduced or enlarged accordingly. This percentile curve is used on the products in the mold work to select the appropriate dimensions.

The outer layer (1) can be made of polyethylene or polypropylene plastic or any other suitable material.

The inner layer (2) may consist of three sponges of different thicknesses or any other suitable material. In an exemplary embodiment of the inventive cranial and protective helmet; the first sponge is the sponge to be used between 4-7 months. The second sponge is the sponge to be used between 8-11 months. The third sponge is the sponge to be used between 12-18 months. All three of these sponges will be useful both for size adjustment and to prevent the plastic layer outside from damaging the baby's head.

The advantages obtained with the developed cranial and protective helmet are listed below.
- The fact that the inner layers (2) of the helmet are of varying thicknesses and varying numbers, can be put on and taken off optionally, and that the thickness or number can be reduced as the skull grows, allows the dimension / size to be enlarged, eliminating the need for appropriate helmet design and individualization according to the growing skull structure.
- In customized productions, it was necessary to take measurements for each baby with plaster or 3D scanning method. Thanks to the developed cranial and protective helmet, the need for taking measurements has been eliminated with a helmet suitable for mass production.
- The cost was reduced with the developed helmet. Due to the very high cost of helmets available in the sector, the use of helmets in mild cases is generally low. With the reduction of helmet costs, it is expected that the use of helmets will increase at levels where the degree of skull deformity is mild. In this way, it will be seen that treatment effectiveness increases and better results are achieved.
- After determining a general outer mold according to the normal baby head size, the thickness of the outer layer (1), inner layers (2) and intermediate layers (3) are determined. The adjustment mechanism (6) was used to adjust the size and dimension for each baby. Thus, there is no need to take measurements for each baby separately and to produce helmets in different sizes.
- The inner layer (2) is washable, removable, hygienic, modular and breathable. The inner layer (2) can be made sweat absorbent thanks to the laminated fabric (4). Various properties can be provided by applying surface coating materials (8) with antibacterial and antimicrobial, deodorizing or moisturizing properties to the inner layer (2).
- Application to fast and mass production as desired with single or multi-core molds is ensured. In this way, the production speed increases and all the preliminary work done before production has been eliminated.

Embodiments of the present invention may be understood by reference to the exemplary embodiments depicted in the drawings. However, the drawings only illustrate typical embodiments of the present invention. It should be noted that since the invention may allow other equally effective embodiments, it will not be presumed to limit its scope.

To facilitate understanding, identical reference numbers have been used where possible to indicate identical elements common to the figures. The figures are not drawn to scale and can be simplified for clarity. It is considered that elements and features of one application can be usefully incorporated into other applications without the need for further explanation.

## Claims

1. The invention relates to a cranial and protective helmet for the correction of cranial deformities comprising
- an outer layer (1) that acts as a mold for the helmet and is modular and has a perforated and/or non-perforated structure,
- an inner layer (2) on the inner surface of the helmet, compatible with the outer layer (1) and that is perforated, breathable, soft, single or multi-layered, or of different thicknesses,
- an intermediate layer (3) between the outer layer (1) and the inner layer (2), consisting of a mesh, perforated structure and a thin plastic barrier,
parts and **is characterized in that** it comprises;
- fastening materials (5) between the outer layer (1), the inner layer (2) or the intermediate layers (3), for fixing the outer layer (1) and the intermediate layer (3), the inner layer (2) and the intermediate layer (3), multiple inner layers (1) and the outer layer (1) and the inner layer (2) to each other,
- an adjustment mechanism (6) on the outer or inner surface of the helmet, which allows it to be assembled and secured, and for size/fit adjustment by means of millimetric adjustment marks,
- joining parts (7) which are located on the right, left, front and rear parts of the outer layer (1) of the helmet and which are modular and which enable the outer layer (1) parts and all parts to be joined together
elements.

2. The invention relates to a cranial and protective helmet for correction of cranial deformities as in claim 1, **characterized in that** it comprises a laminated fabric (4) which can be applied to the surface of the outer layer (1), the inner layer (2) or the intermediate layers (3) to absorb sweat, contribute to washability and reduce irritation.

3. The invention relates to a cranial and protective helmet for the correction of cranial deformities as in claim 1 or claim 2, **characterized in that** the fastening materials (5) are means for securing the outer layer (1), the inner layer (2) and the intermediate layers (3) to each other.

4. The invention relates to a cranial and protective helmet for correction of cranial deformities as claimed in any one of the preceding claims, **characterized in that** it comprises surface coating materials (8) having moisturizing, regenerating, deodorizing or bactericidal properties applied to the inner layer (2) of the helmet.

5. The invention relates to a cranial and protective helmet for correction of cranial deformities as claimed in any one of the preceding claims, **characterized in that** it comprises a chin strap (9) connected to the outer layer (1), which allows the helmet to be secured to the chin when the helmet is worn.

6. The invention relates to a cranial and protective helmet for the correction of cranial deformities as claimed in any one of the preceding claims, **characterized in that** it comprises a chin strap adjuster (10) on the chin strap (9) for adjusting the chin strap (9).

7. The invention relates to a cranial and protective helmet for correction of cranial deformities as claimed in any one of the preceding claims, **characterized in that** the outer layer (1), inner layer (2) and intermediate layers (3) of the helmet are mass-produced by injection moulding.
